# EUROPEAN PATENT APPLICATION

(11) **EP 4 570 167 A1**
(43) Date of publication of application: **18.06.2025**
(21) Application number: 23217045.6
(22) Date of filing: 15.12.2023
(51) Int. Cl.: A61B 5/00, A61B 5/16, G10L 25/00, G16H 50/00

(54) **DETERMINING PSYCHOLOGICAL FACTORS OR METRICS FROM USER SPEECH DATA**

(71) Applicant: Thymia Limited, London N1 7SR (GB)
(72) Inventor: Goria, Stefano, London, SE8 3GR (GB); Molimpakis, Emilia, London, NW10 5PT (GB); Georgescu, Alexandra-Livia, London, E3 4TX (GB)
(74) Representative: Bryers Intellectual Property Ltd

(57) **Abstract**

In some examples, an apparatus for generating a set of metrics representing indicators of the psychological state of a user comprises a processor, a memory coupled to the processor, the memory configured to store program code executable by the processor, the program code comprising one or more instructions, whereby to cause the apparatus to generate a set of first audio samples from user audio data, wherein each audio sample of the set of first audio samples comprises a segment of natural speech determined from the user audio data, generate a set of second audio samples from the user audio data, wherein each audio sample of the set of second audio samples comprises a segment of audio representing a phoneme determined from the user audio data, generate, using the set of first audio samples, a set of first embeddings comprising respective vector representations for each audio sample of the set of first audio samples, generate, using the set of second audio samples, a set of second embeddings comprising respective vector representations for each audio sample of the set of second audio samples, generate, using the set of first embeddings and the set of second embeddings, respective measures of probability for each symptom of a set of multiple symptoms for the user, and using the probability measures, calculate the set of metrics.

## Description

### Technical Field

The present disclosure relates, in general, to a method for detecting, e.g., fatigue/exhaustion, stress, distress, sleep propensity and confidence from user voice recordings.

### Background

Diagnostic scales like the PHQ-9 for depression and GAD-7 for anxiety offer precise measures of mental health aspects. Yet, they are not ideal for digital mental health and wellness platforms for two main reasons: Firstly, questionnaires are subjective and can be willingly influenced; second, extensive negative and problem-focused language as characteristic for screening and diagnostic questionnaire items can deter user engagement, especially for healthier users. Finally, questionnaire fatigue is known to set in when asking participants to fill out questionnaires after questionnaires, some with more than 30 items. Other more objective methods, like electroencephalography require specialized instruments, are often expensive, intrusive, and may not be easily integrated into daily routines or provide real-time feedback. They would also only cover a limited subset of relevant wellness indicators, e.g. tiredness/sleepiness.

Machine learning, with its promise of automation and precision, has been eyed as a potential solution to the challenge of detecting and measuring wellness indicators. However, many current models have limitations stemming from, e.g., small sample sizes or a narrow time frame for assessment. Such constraints diminish their overall validity, accuracy, and sensitivity.

Therefore, there exists a clear and pressing need for innovative wellness detection solutions that are universally applicable and adaptable to diverse real-world contexts. Methods that can be seamlessly incorporated into people's daily lives, such as voice-based fatigue detection, could potentially bridge this gap. These approaches, offering real-time, non-intrusive, and user-centric solutions, could drastically reduce fatigue-induced incidents, promoting both safety and optimal performance across various sectors.

### Summary

An objective of the present disclosure is to provide a set of metrics representing indicators of the psychological state of a user.

The foregoing and other objectives are achieved by the features of the independent claims.

Further implementation forms are apparent from the dependent claims, the description and the Figures.

A first aspect of the present disclosure provides an apparatus for generating a set of metrics representing indicators of the psychological state of a user, the apparatus comprising a processor, a memory coupled to the processor, the memory configured to store program code executable by the processor, the program code comprising one or more instructions, whereby to cause the apparatus to generate a set of first audio samples from user audio data, wherein each audio sample of the set of first audio samples comprises a segment of natural speech determined from the user audio data, generate a set of second audio samples from the user audio data, wherein each audio sample of the set of second audio samples comprises a segment of audio representing a phoneme determined from the user audio data, generate, using the set of first audio samples, a set of first embeddings comprising respective vector representations for each audio sample of the set of first audio samples, generate, using the set of second audio samples, a set of second embeddings comprising respective vector representations for each audio sample of the set of second audio samples, generate, using the set of first embeddings and the set of second embeddings, respective measures of probability for each symptom of a set of multiple symptoms for the user, and using the probability measures, calculate the set of metrics.

In an implementation of the first aspect, the program code can comprise one or more instructions, whereby to cause the apparatus to generate a set of third embeddings by concatenating respective ones of the vector representations for each audio sample of the set of first audio samples with a corresponding vector representation for each audio sample of the set of second audio samples. The program code can comprise one or more instructions, whereby to cause the apparatus to calculate, using the set of second audio samples, respective F 1 formant time series and respective F2 formant time series for each phoneme determined from the user audio data, and use the F1 formant time series and the F2 formant time series to generate a set of two dimensional path signatures for respective ones of the phonemes determined from the user audio data. The program code can comprise one or more instructions, whereby to cause the apparatus to combine the probability measures according to a predefined set of profiles comprising additive and/or multiplicative rules for the probability measures.

In an example, the program code can comprise one or more instructions, whereby to cause the apparatus to compare respective measures of probability for each symptom of a set of multiple symptoms for the user with a set of benchmark values to generate a benchmarked probability score for each symptom.

A second aspect of the present disclosure provides a machine-readable storage medium encoded with instructions for generating a set of metrics representing indicators of the psychological state of a user, the instructions executable by a processor, whereby to cause the processor to generate a set of first audio samples from user audio data, wherein each audio sample of the set of first audio samples comprises a segment of natural speech determined from the user audio data, generate a set of second audio samples from the user audio data, wherein each audio sample of the set of second audio samples comprises a segment of audio representing a phoneme determined from the user audio data, generate, using the set of first audio samples, a set of first embeddings comprising respective vector representations for each audio sample of the set of first audio samples, generate, using the set of second audio samples, a set of second embeddings comprising respective vector representations for each audio sample of the set of second audio samples, generate, using the set of first embeddings and the set of second embeddings, respective measures of probability for each symptom of a set of multiple symptoms for the user, and using the probability measures, calculate the set of metrics.

In an implementation of the second aspect, the machine-readable storage medium can further comprise instructions executable by the processor, whereby to cause the processor to generate a set of third embeddings by concatenating respective ones of the vector representations for each audio sample of the set of first audio samples with a corresponding vector representation for each audio sample of the set of second audio samples. The machine-readable storage medium can further comprise instructions executable by the processor, whereby to cause the processor to calculate, using the set of second audio samples, respective F 1 formant time series and respective F2 formant time series for each phoneme determined from the user audio data, and use the F 1 formant time series and the F2 formant time series to generate a set of two dimensional path signatures for respective ones of the phonemes determined from the user audio data. The machine-readable storage medium can further comprise instructions executable by the processor, whereby to cause the processor to combine the probability measures according to a predefined set of profiles comprising additive and/or multiplicative rules for the probability measures.

In an example, the machine-readable storage medium can comprise instructions executable by the processor, whereby to cause the processor to compare respective measures of probability for each symptom of a set of multiple symptoms for the user with a set of benchmark values to generate a benchmarked probability score for each symptom.

A third aspect of the present disclosure provides a method for generating a set of metrics representing indicators of the psychological state of a user, the method comprising generating a set of first audio samples from user audio data, wherein each audio sample of the set of first audio samples comprises a segment of natural speech determined from the user audio data, generating a set of second audio samples from the user audio data, wherein each audio sample of the set of second audio samples comprises a segment of audio representing a phoneme determined from the user audio data, generating, using the set of first audio samples, a set of first embeddings comprising respective vector representations for each audio sample of the set of first audio samples, generating, using the set of second audio samples, a set of second embeddings comprising respective vector representations for each audio sample of the set of second audio samples, generating, using the set of first embeddings and the set of second embeddings, respective measures of probability for each symptom of a set of multiple symptoms for the user, and using the probability measures, calculating the set of metrics.

In an implementation of the third aspect, the method can further comprise generating a set of third embeddings by concatenating respective ones of the vector representations for each audio sample of the set of first audio samples with a corresponding vector representation for each audio sample of the set of second audio samples. The method can further comprise calculating, using the set of second audio samples, respective F 1 formant time series and respective F2 formant time series for each phoneme determined from the user audio data, and using the F1 formant time series and the F2 formant time series, generating a set of two dimensional path signatures for respective ones of the phonemes determined from the user audio data.

In an example, the method can further comprise combining the probability measures according to a predefined set of profiles comprising additive and/or multiplicative rules for the probability measures. The method can further comprise comparing respective measures of probability for each symptom of a set of multiple symptoms for the user with a set of benchmark values to generate a benchmarked probability score for each symptom.

These and other aspects of the invention will be apparent from the embodiment(s) described below.

### Brief Description of the Drawings

In order that the present disclosure may be more readily understood, embodiments will now be described, by way of example, with reference to the accompanying drawings, in which:
Figure 1 is a schematic representation of a system according to an example;
Figure 2 is a schematic representation of part of the system of figure 1, according to an example;
Figure 3 is a schematic representation of user audio data, according to an example; and
Figure 4 is a schematic representation of a machine according to an example.

### Detailed Description

Example embodiments are described below in sufficient detail to enable those of ordinary skill in the art to embody and implement the systems and processes herein described. It is important to understand that embodiments can be provided in many alternate forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

The terminology used herein to describe embodiments is not intended to limit the scope. The articles "a," "an," and "the" are singular in that they have a single referent, however the use of the singular form in the present document should not preclude the presence of more than one referent. In other words, elements referred to in the singular can number one or more, unless the context clearly indicates otherwise. It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof. The term "and/or" is only an association relationship for describing associated objects and represents that three relationships may exist such that A and/or B may indicate that A exists alone, A and B exist at the same time, or B exists alone. The character "/" generally represents that the associated objects are in an "or" relationship.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

The following contains specific information related to implementations of the present disclosure. The drawings and their accompanying detailed disclosure are merely directed to implementations. However, the present disclosure is not limited to these implementations. Other variations and implementations of the present disclosure will be obvious to those skilled in the art.

The phrases "in one implementation," or "in some implementations," may each refer to one or more of the same or different implementations. The term "coupled" is defined as connected whether directly or indirectly through intervening components and is not necessarily limited to physical connections. The expression "at least one of A, B and C" or "at least one of the following: A, B and C" means "only A, or only B, or only C, or any combination of A, B and C."

The terms "system" and "network" may be used interchangeably.

For the purposes of explanation and non-limitation, specific details such as functional entities, techniques, protocols, and standards are set forth for providing an understanding of the present disclosure. In other examples, detailed disclosure of well-known methods, technologies, systems, and architectures are omitted so as not to obscure the present disclosure with unnecessary details.

Persons skilled in the art will immediately recognize that any network function(s) or algorithm(s) disclosed may be implemented by hardware, software or a combination of software and hardware. Disclosed functions may correspond to modules which may be software, hardware, firmware, or any combination thereof.

A software implementation may include machine- and/or computer- readable and/or executable instructions stored on a machine- and/or computer-readable medium such as memory or other types of storage devices. One or more microprocessors or general-purpose computers with communication processing capability may be programmed with corresponding executable instructions and perform the disclosed network function(s) or algorithm(s).

The microprocessors or general-purpose computers may include Applications Specific Integrated Circuitry (ASIC), programmable logic arrays, and/or using one or more Digital Signal Processor (DSPs). Although some of the disclosed implementations are oriented to software installed and executing on computer hardware, alternative implementations implemented as firmware or as hardware or as a combination of hardware and software are well within the scope of the present disclosure. The computer readable medium includes but is not limited to Random Access Memory (RAM), Read Only Memory (ROM), Erasable Programmable Read-Only Memory (EPROM), Electrically Erasable Programmable Read-Only Memory (EEPROM), flash memory, Compact Disc Read-Only Memory (CD-ROM), magnetic cassettes, magnetic tape, magnetic disk storage, or any other equivalent medium capable of storing computer-readable instructions.

The state of mental and emotional well-being is paramount in determining an individual's overall health, capacity to perform, and quality of life. Traditional understanding has often reduced the notion of wellness to mere physical health. However, contemporary perspectives are broadening this scope, recognizing that true wellness encapsulates a diverse array of both mental and emotional indicators.

According to an example, there is provided a method and apparatus for generating a set of metrics representing indicators of the psychological state of a user. The set of metrics can provide one or more wellness scores for the user that can be used to provide a quantitative and/or qualitative measure representing any one or more of fatigue/exhaustion/burnout, sleep propensity, distress and/or stress, self-esteem/confidence of the user. For example, beyond the traditional understanding of fatigue stemming from lack of sleep, exhaustion or burnout is a multidimensional phenomenon that arises from sustained periods of cognitive or physical exertion, potentially compromising both well-being and performance. Sleep propensity, such as the inclination to feel drowsy or fall asleep is not merely about how much one slept the previous night. It comprises a complex measure that might hint at underlying sleep disorders or other health concerns. Distress and stress may seem similar, but they cater to unique aspects of emotional suffering. Distress revolves around the emotional and psychological inability to cope or adapt to challenging situations, while stress corresponds to physiological reactions to perceived threats or challenges. Self-Esteem/Confidence represents a cornerstone of psychological well-being, this metric addresses one's self-worth, belief in their capabilities, and how they perceive themselves in relation to external expectations.

Given the intricate web of these indicators, it is evident that a holistic approach to wellness detection is essential. Traditional methods often fall short, being either too subjective or intrusive, and not easily adaptable to daily life.

In an example, by utilising audio samples from user audio data, such as voice recordings for example, with the option to use, e.g., metadata such as sleep patterns, it is possible to generate comprehensive indicators related to emotional arousal, valence, exhaustion, distress, and more. Moreover, new and past recordings can be used to continually refine insights, ensuring a dynamic and individual-specific assessment. A voice-based detection mechanism according to an example can provide accuracy and seamless integration into daily routines, making wellness tracking more accessible and user-friendly than ever before.

According to an example, at least some of the above set of mental wellness scores can be derived from predefined combinations of underlying observable mental health symptoms that can be measured from speech samples. In an example, a set of machine learning models, such as discriminative predictive models, can be used to estimate mental health symptoms from speech. A discriminative predictive model refers to a type of model in machine learning that focuses on distinguishing between different categories or classes based on input data. It models the conditional probability P(y|x) , where y is the output or label and x is the input data. The goal is to find the boundary or decision surface that best separates the classes in the input space. Regression models, where y is a continuous output and x is the input data, can also be used.

In general, a machine learning model is defined by a "model architecture", a "training dataset" and a "cost function". The architecture is the set of mathematical operations that transform input to output, the cost function encodes a metric that needs to be minimised for the model to perform correctly and the training dataset is a collection of input-output pairs where the output is considered "ground truth" i.e. the output that we wish the model would be able to produce for that given input. In the present disclosure, there are two primary input components: a media recording and its accompanying metadata. In an example, a media recording comprises user audio data (i.e., audio data comprising a recording of user speech). In some examples, the audio data can comprise at least 20 seconds, or thereabouts, of human (i.e., the user's) speech. Any suitable file format may be used for the audio data. For example, audio data may be provided as MPEG-4 (MP4), Web Media File (WEBM), MPEG Audio Layer III (MP3), Ogg, WAV, AVI, and MOV, and so on. In an example, audio data comprises an audio recording at a sample rate of at least 16kHz.

According to an example, metadata associated with audio data comprises information that provides contextual information about the subject (user) and the recording. For example, metadata associated with audio data for a user can comprise, at least one of the following: Age: the current age of the subject, e.g., in years, birth sex: such as male, female, or intersex for example, language: the language primarily used by the subject during the recording. Any language may be supported, providing that, for example, it is a language for which a viable automatic speech recognition (ASR) engine is available, and for which a viable pronunciation dictionary is available.

Input audio data can be transformed into a standardised format for further processing. For example, an input recording can be converted to an audio-only, mono channel, 16kHz wav format audio data file. Should the input file be corrupted or, e.g., incompatible with anticipated output specifications, an error-handling mechanism can be implemented. In an example, an exception can be triggered in the event that an input file is corrupted or incompatible, thereby halting any further processing steps and communicating the nature of the discrepancy to a system user. As an example, a recording not meeting a baseline criterion, such as an audio track recorded below 16kHz frequency or an audio track shorter than 20 seconds for example, can prompt an early-stage exception. Subsequent phases, as described in more detail below, can conduct more granular analysis of, e.g., speech duration. Existing packages, such as 'ffmpeg' for example, can be used for recording and/or converting and/or almost all other manipulations of audio data.

For example, the following command can be employed:
ffmpeg -i inputfile.ext -ac 1 -ar 16000 -acodec pcm _s 16le outputfile.wav in which:
i. '-i inputfile.ext': Specifies the path to the input file
ii. '-ac 1': Converts audio to mono (single channel)
iii. '-ar 16000': Sets the audio sample rate to 16kHz
iv. '-acodec pcm_s16le': Designates the audio codec to be used, ensuring it is a 16-bit PCM wav format
v. 'outputfile.wav': The name of the output file in .wav format

According to an example, a Speech-to-Text (STT) conversion engine can be used to transform raw audio content (in the form of raw audio data) into readable transcripts, serving as a foundation for subsequent analysis and interpretation. Typical input specifications can be of the form:
- Format: .wav
- Frequency: 16kHz
- Channel: Mono
- Additional Info: Language spoken in the recording

According to an example, a system as described herein can interface with a variety of Automatic Speech Recognition (ASR) or speech to text (STT) providers, be they third-party solutions or proprietary in-house systems. The primary criteria for compatibility include support for the spoken language in the recording, provision of word-level timestamps, availability of confidence scores for each recognized word, and existence of an API tailored for batch transcription (no real-time or streaming). For example, upon transcription, results from various ASR solutions can be harmonised into a singular, provider-agnostic format. This streamlines subsequent analytical processes and ensures consistency. Supported Third-Party Providers may include, e.g., AWS Transcribe, Google STT, Azure STT, Deepgram, Assembly.ai, Speechgram, Whisper. To ensure uniformity, all transcription results can be reformatted to comply with specific formats. Examples, in the form of Python data classes, are as follows:
@dataclass
class SpeechToTextItem:
   start_time: float
   end_time: float
   word: str
   confidence: float
@dataclass
class SpeechToTextResults:
   transcript: str
   items: List[SpeechToTextItem]
   source: str

In the event that a transcription encounters an issue, an error handling mechanism can be used to trigger an exception that can implement a pause in processing audio data. A detailed notification can be dispatched, informing relevant stakeholders of the specific stage and nature of the failure.

According to an example, a set of first audio samples can be generated from user audio data. Each audio sample of the set of first audio samples can comprise a segment of natural speech determined from the user audio data, also referred to as an utterance. Similarly, a set of second audio samples can be generated from the user audio data. Each audio sample of the set of second audio samples can comprise a segment of audio representing a phoneme determined from the user audio data.

An audio sample may be referred to as an audio cut, in either case defining a fragment of an audio recording from a user. Generally speaking, audio gets 'cut 'into segments comprising smaller audio files to maximise the signal that can be extracted by a machine learning model.

According to an example, an utterance comprises a fragment or snippet of audio from continuous speech. Utterances can include short pauses, but typically an utterance comprises one sentence; they therefore capture/represent natural speech. Phonemes comprise shorter fragments or snippets of audio which capture regions of speech comprising perceptually distinct units of sound (in a specified language) that distinguish one word from another. Example phonemes that provide particularly information-rich units of sound are vowels; these are known to be affected by physical, cognitive and mental health conditions that can affect speech production.

In an example, an"utterance can comprise a segment of speech which contains pauses or silences not greater than about 0.5 seconds. Generally, silences can be characterised as follows:
- Micropause (silence of 0.2s or less)
- Hesitation (silence of 0.3 to 0.4s)
- Unfilled pause (silence of 0.5s or more)

According to an example, in order to identify where in audio data comprising an audio recording there is speech or silence a SpeechToText output can be used which can include time-stamps of the beginning and end of every spoken word. Subsequently, transcribed words and short pauses (e.g., less than 0.5 seconds) can be merged together and the results aligned to the actual audio content. The pair of an audio file with its corresponding transcription file with start and end times defines a "supervision" file forming an aligned recording that can be used to create audio cuts.

Configuration parameters for generating a set of first audio samples from user audio data can be as follows:
- segment_length; duration of an utterance; default value is 20 seconds
- Overlap_length; utterances can be created as completely independent segment of audio or partly overlapping ones, this parameter from 0 to segment_length drives the amount of overlap; useful to generate more samples from the same recording
- Max_silence; maximum silence before cutting the recording; default value is 0.5 seconds
- Silence_padding; amount of silence that gets padded at the beginning and the end of every utterance, default value is 0.2 seconds

The process to create an utterance cut from an aligned recording is, in an example:
1. trim trailing silences from the beginning and the end of the recording; typically, there is a long silence before people start talking in most common protocols to collect speech data, similarly there might be an unnatural long silence at the end where people are stopping the recording. These are removed as they are characteristics of the methods used to collect speech as opposed to being linked to signals of interest.
2. Cut the recording where there are pauses longer than 0.5 seconds; this gives as a result a list of recordings which only contain speech or micropauses and hesitations.
3. Append, on both sides of the recordings, a small amount of silence according to a parameter extra_silence_gap (e.g., 0.2 seconds).
4. Merge the list of recordings into a longer recording by appending every recording to the previous one.
5. Cut into windows of equal duration the merged recording (default duration is 20 seconds); the windows can overlap according to the parameter overlap_length
6. Place each utterance cut in a separate audio file with a name that reports the UUID of the original recording, basic configuration of the utterance design (duration, overlap) and an incremental counter, e.g., UUID_20s_2s_0.wav for the first 20 seconds cut with 2 seconds overlap in the recording UUID.

A set of second audio samples generated from user audio data comprises individual, distinct speech sound units that make up (and are able to distinguish within the given language) words. That is, the set of second audio samples comprise phonemes. According to an example, a collection of short recording segments can be generated from the input audio data and each labelled against a list of predefined phonemes.

As such, inputs are:
- Audio data, e.g., a .wav file
- A list of desired phonemes
- A supervision file with timestamps aligned to each word
- A pronunciation dictionary for the language spoken in the recording For the below we assume that a pronunciation dictionary for the relevant language is available in the format of txt files as:
   iso_code.txt

Where iso_code is the ISO language code, and every row of the file is in the format:

### [ENTRY] [TAB] [IPA]

For example for English UK we have for the word "sheep" the following:
sheep /∫'i 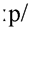 p/

For any given language (defined additionally by geographical region to capture distinct language dialects), a complete list of said language's phonemes can be compiled. An example of the list of desired phonemes for English UK is given by the below python dataclass:
class UKVowelsSet(Enum):
KEY= "'i 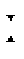 "
SHIP = "'_{I}"
DRESS = "'ε"
TAP = "'æ"
CROSS = "' 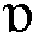 "
THOUGHT = "' 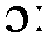 "
FOOT = "'u"
GOOSE = "'u 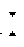 "
CUP = "'∧"
SPA = "' 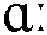 "
EARTH = "' 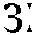 "
FACE = "'e 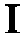 "
KNIFE = "'a 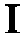 "
BOY = "' 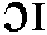 "

Where the 14 English vowels are selected and labelled with a typical word which contains them. Herein, a specific vowel will be referred to by using its corresponding prototype word: e.g., instead of " ' 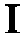 " the word SHIP (all uppercase letters) will be used.

According to an example, a set of second audio samples can be generated from user audio data as follows:
1. Take the transcript with time stamp associated for each word and substitute each word with the corresponding vowel label; for example, the words "key" and "lethal" will be both substituted with the label KEY.
2. Words that have no match substituted with the label NONE.
3. Cut the audio file in correspondence with the start and end of vowel labels.
4. Pad with silence at the beginning and end of each cut according to the parameter Silence_padding.
5. Place each audio fragment in a separate smaller file named after the occurrence of a vowel label with an indication of the padding and incremental counter after prepending the UUID of the original recording, e.g., UUID_KEY_10ms_0.wav, UUID_KEY_10ms_1.wav, UUID_B O Y_ 10m s_0. wav, etc.

At the end of this step the system has produced a set of intermediate audio files and associated meta-data ready for further processing.

According to an example the set of first audio samples can be used to generate a set of first embeddings comprising respective vector representations for each audio sample of the set of first audio samples. The set of second audio samples can be used to generate a set of second embeddings comprising respective vector representations for each audio sample of the set of second audio samples. Embeddings comprise a numerical representation in vector space of different input types. In the present context, the input is a collection of audio files (i.e., the set of first audio samples and the set of second audio samples). Different classes of embeddings can be generated depending on the cut type applied to them.

When observed at different scales, different nuanced pieces of information are available in speech, e.g., utterances (audio segments of natural speech of a duration of 20 seconds) and phonemes (comprised in audio having durations of generally less than around 1 second to 1 second). [.] Phoneme-level audio cuts, by containing a single phoneme, allow for a much more focused assessment of those individual speech sounds that may be affected by - and thereby connected to - changes in the speaker's physical, cognitive or mental health state and which can cause variation in the speaker's speech production.

According to an example, utterance level embeddings comprise a map from a fixed duration speech cut to a fixed size numerical vector. An embeddings model can comprise a compression algorithm that compresses an input comprising 16000 x 20 = 320000 numbers (i.e., audio data comprising a 20 second audio sample at 16 kHz) to an output vector comprising 1024 numbers. In an example, the resulting output vector can also comprise rich information about speech production, making it possible for downstream models to specialise and provide information about physical and mental states of the speaker.

In the case of utterance level embeddings, the following can apply, according to an example:
- Input of fixed duration speech at 16kHz sampling rate
- Output comprising a vector of floating numbers of fixed size; example sizes are in form of 2^{x} with x an integer between 8 and 12
- Can be applied as a starting point for shallow downstream models in paralinguistic (i.e., relating to non-lexical communication components) tasks against popular benchmark datasets (available for example at https://www.tensorflow.org/datasets/catalog/overview#audio) with performance at least in line with that noted in the table below; shallow models that are acceptable as a benchmark are Logistic Regression and Support Vector Machines.
- The benchmark dataset must not be part of the training data of the embedding model.

| Dataset | Task | Minimum Accuracy |
|---|---|---|
| CREMA-D | Emotion recognition | 0.67 |
| VoxForge | Language Identification | 0.88 |
| SpeechCommands | Commands recognition | 0.74 |
| SAVEE | Emotion recognition | 0.67 |
| DementiaBank | Dementia/healthy | 0.67 |

Example models available for commercial use that fit the above requirements are, e.g.:
- TRILL - https://www.kaggle.com/models/google/nonsemantic-speech-benchmark/frameworks/tensorFlow2/variations/trill
- TRILLSON - https://www.kaggle.com/models/google/trillsson
- WAV2VEC2 - https://huggingface.co/docs/transformers/model_doc/wav2vec2

Note that TRILL and WAV2VEC2 do not natively output a vector of fixed size, but an array of fixed size vectors; in those cases, the compatible version of the model is one that appends an average pooling layer after the raw output of the embeddings model.

According to an example, phoneme level embeddings can be created by:
1. Computing F1 and F2 formant time series for the audio segment corresponding to a specific phoneme
2. Extracting the path signatures up to order 2 of the 2-dimensional time series in the F1 and F2 space
3. Combining the output of the N formants path signatures into a NxP matrix where P is the number of signatures extracted.

A formant is a concentration of acoustic energy around a particular frequency in a speech wave. They comprise distinctive frequency components of the acoustic signal produced by speech. There are several formants, each at a different frequency, roughly one in each 1000Hz band for average men, for example. The corresponding range for average women is one formant every 1100Hz. The true range depends on the actual length of the vocal tract. Each formant corresponds to a resonance mode of the vocal tract.

By convention, oral formants are numbered consecutively upwards from the lowest frequency. Even though theoretically higher order formants would be interesting, the actual estimation tends to be more noisy and so, in an example, formant time series up to (and including) F2 are considered.

Different methods exist to obtain formant information. Formant frequencies, in their acoustic definition, can be estimated from the frequency spectrum of the sound, using a spectrogram or a spectrum analyzer for example. To estimate the acoustic resonances of the vocal tract (i.e., the speech definition of formants) from a speech recording, linear predictive coding can be used and which may be implemented in software such as, e.g., Praat. Another approach comprises extracting a spectral envelope by neutralizing the fundamental frequency and only then looking for local maxima in the spectral envelope. According to an example, path signatures provide a robust mathematical framework capable of capturing intricate temporal dependencies and patterns in sequential data. An extensive review in "Signature Methods in Machine Learning", Terry Lyons & Andrew D. McLeod, January 27, 2023, outlines their application in estimating gesture correctness, predicting bipolar disorder, Alzheimer's disease, or early sepsis, as well as in decision-making, speech emotion recognition, and analysis of medical prescriptions.

According to an example, path signatures are applied to summarise formant information. For example, the time series of the formant time series F1 and F2 can be represented as a path in 2 dimensions. Path signatures can be constructed as iterated integrals of the path, capturing non-linear features of the data.

Observable symptoms models (OSMs) can be implemented, and can comprise discriminative models that can, for a given speech input that has been pre-processed by specific embeddings models, output the probability of a specific symptom being present or the severity of the same.

For example, using pre-computed utterance level and phoneme level embeddings as inputs, OSMs can be used to output an estimate for any one or more of: Patient Health Questionnaire (PHQ) PHQ_1 to PHQ_8 symptoms (a shortened but widely used version of the PHQ_9 that excludes questions on suicidality and self-harm), the PHQ-8 can be used as a measure of current depression in the general population. Journal of affective disorders, Kroenke, K., Strine, T. W., Spitzer, R. L., Williams, J. B., Berry, J. T., & Mokdad, A. H. (2009), 114(1-3), 163-173; Generalized Anxiety Disorder Screener (GAD) GAD_1 to GAD_7 symptoms (Spitzer, R. L., Kroenke, K., Williams, J. B. W., & Löwe, B. (2006). Generalized anxiety disorder 7 (GAD-7)[Database record]. APA PsycTests.); a fatigue survey (FS) consisting of three items (FS_1 "I find myself getting tired easily", FS_2 " My fatigue hinders my ability to engage in sustained physical activities" and FS_3 "Fatigue makes it difficult to do work, take care of things at home or do things with other people"), perceived time from sleep and "objectively fatigued class probability". According to an example, multiple independent models can be used to predict the scores from the additive observables and multiplicative observables defined in the table below. In an example, the groups of observables that constitute the additive observables are normalised to be bounded in the range of values [0,1].

| **SCORE** | **ADDITIVE OBSERVABLES** | **MULTIPLICATIVE OBSERVABLES** |
|---|---|---|
| **EXHAUSTION/ FATIGUE** | fs_1, fs_2, fs_3, phq4 | 1 |
| **SLEEPINESS** | perceived_time_from_sleep | 1 |
| **DISTRESS** | phq 1, phq2, phq6, gad 1, gad 2,gad 3, gad 7 | objectively_fatigued_score |
| **STRESS** | phq3, phq4, phq5, phq7, phq8, gad 4, gad 5, gad 6 | objectively_fatigued_score |
| **CONFIDENCE** | phq8, fs-3, fs-2 | 1 |

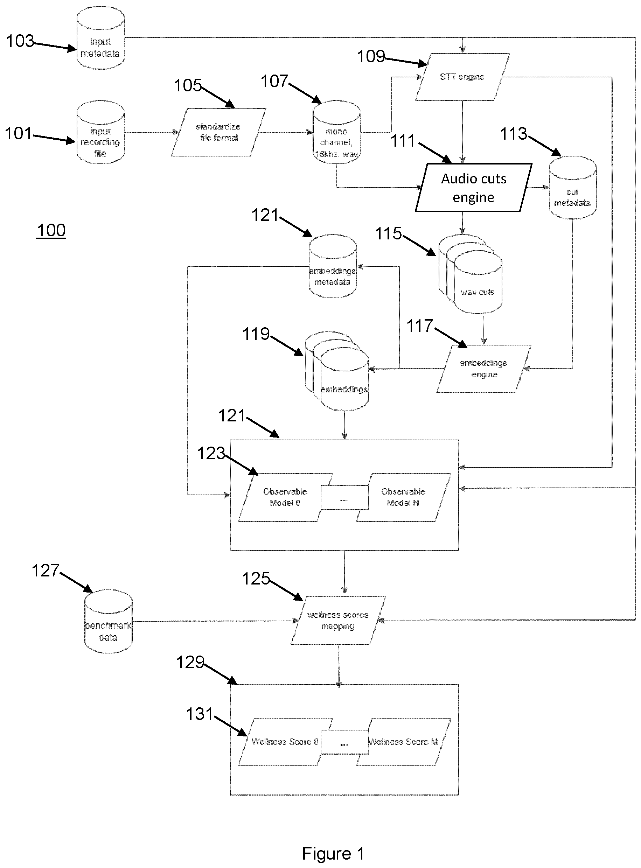

From the table above we can identify 6 Observables Models, the output of which can be combined to produce five scores. In particular, in this example we have the output of OSM_1 being the sum of the Additive Observable in the first row of the table, normalised in the range [0,1]; similarly we define the targets for OSM_2, ... OSM_5 while the target for the final model OSM_6 is given by the "objectively_fatigued_score". The 5 scores "fatigue", "sleepiness", "distress", "stress" and "confidence" are defined as the product of the OSM output from additive observables and multiplicative observables. For example, "fatigue" is the product of the output of OSM_1 and 1, while "stress" is the product of OSM_4 and OSM_6. The six models noted in the table above share the same architecture which is described in more detail below with reference to figure 1. In an example, n phoneme-level embeddings (with n the number of selected phonemes) are fed through a sequence of Convolution Layers and BatchNorm layers and then concatenated with the utterance level embeddings, as described above. Subsequent layers can be, e.g., dense fully connected layers with, e.g., ReLU activation functions.

According to an example, the problem can be framed as a regression problem for all scores but the "objectively fatigued class probability"; therefore, for the first 5 models a RMSE loss function can be used, while for the last model a logit cross entropy function which is more suitable for a classification problem can be used. All scores can be finally trimmed in the space [0,1].

Wellness scores can be obtained by combining a set of outputs from the OSMs. For example, additive components can be added together and finally multiplied by multiplicative components. The breakdown for each score according to an example is provided in the above table.

Finally, before producing an output, the raw value of the scores can be compared against a benchmark dataset. Age, birth sex and the spoken language of the user can be provided. Using these data, for each age-sex-language group, a histogram of raw data from a benchmark dataset is determined and by determining the quantile of the raw score the final output value for each of 5 dimensions can be assigned, for example:
- Low - if the score is in the low 25 percentile of the corresponding benchmark group
- Medium-low - if the score is in between the 25 and 50 percentile of the corresponding benchmark group
- Medium-high - if the score is in between the 50 and 75 percentile of the corresponding benchmark group
- High - if the score is above the 75 percentile of the corresponding benchmark group.

According to an example, a training dataset comprises a curated collection of labelled audio files. Characteristics that are delineated include the nature of the labels, audio quality, diversity of speakers within the recordings, and the aggregate duration of all recordings.

A recording session is defined as a collection of individual recordings for a speaker where the speaker is asked to record their voice while performing a set of simple tasks. In an example, a session can comprise the following tasks:
Task 1 - picture description:
   The speaker is asked to describe what they see in a busy picture; the picture can contain a high number (e.g., at least 20) of entities (people, animals, objects etc.) and depict a high number of actions (talking, drinking, walking etc.) in the foreground and background. This constitutes a free speech task aimed at eliciting natural speech. The duration can be at least 30 seconds of speech.
Task 2 - reading task:
   The speaker is asked to read out (aloud) a snippet of text; the text can be phonetically balanced in order to elicit the majority of the most commonly encountered phonemes in the given spoken language (adjusted, if necessary, to account for phonemes encountered in regional dialects). Duration can be at least 30 seconds.
Task 3 - question answering:
   The speaker is asked to respond to a simple question by speaking; the question can be designed to elicit a positive or neutral response including elements of storytelling. Duration can be at least 30 seconds of speech. Example questions: "Tell me about your perfect Saturday", "What is your favourite movie and why".

The same speaker can be engaged in multiple sessions; several distinct instances of the three tasks can be used and the speaker may not be presented twice in a row with the same task. The same task can be used twice in total in the overall collection of sessions.

When collecting the recording data, a set of target data can also be collected for the dataset to be usable within the system. In particular, some target data can be obtained by administering standardised subjective questionnaires, while other targets can be derived from objective measurement. In an example, questionnaires that can be administered are any one or more of: Patient Health Questionnaire - 8 (PHQ-8), which is a shortened version of the PHQ-9, Generalised Anxiety Disorder Questionnaire (GAD-7), Fatigue Survey (FS). The target data can also comprise a measure of time from last sleep (in hours) (where a short nap defined as less than 1 hour of sleep is not considered sleep), a time since finishing the last work session (in hours) (adapted where applicable, e.g., time since finishing the last work session for people with a job, time since finishing university lecture for students etc.), and demographics information containing at least birth year, birth sex and first language for a user.

According to an example, a dataset comprises a collection of speakers of at least, e.g., 1000 individuals, balanced across birth sex, age and language. Multiple recording sessions for each speaker can be used (such as at least 10, for example) collected across a period of time of, e.g., at least 10 days, with half of the recordings collected in a short time window after rest (2 to 4 hours) and half after a work day or just before resting again.

Each recording should comprise at least 30 seconds of speech. In an example, the total minimum number of minutes is: 3 recordings per session (1.5 minutes) multiplied by 10 sessions for 1000 participants = 15000 minutes (250 hours).

Figure 1 is a schematic representation of a system according to an example. The system 100 of figure 1 can be implemented as part of an apparatus for generating a set of metrics representing indicators of the psychological state of a user. In the example of figure 1, an input user audio recording 101 is provided. The input user audio recording 101 comprises a recording of spoken language of a user. For example, the input user audio recording 101 can comprise a recording of user spoken language that has been captured using an audio recording device (not shown) that may be part of another system, such as a smart device (e.g., a smart speaker or smartphone), or a vehicle for example. Input metadata 103 is provided. The input metadata 103 comprises a set of information such as that described above, representing certain characteristics and/or attributes of the user (e.g., age, birth sex, etc.). In an example, the input metadata may also comprise information about a device used to capture the input user audio recording 101, such as information relating to a file format used to store and/or transmit the input user audio recording 101 and/or other details of capture, such as location and/or time of capture etc.

The input user audio recording 101 can be converted to user audio data 105. The user audio data can comprise the input user audio recording 101 in a selected file format, such as .wav for example. For example, the input user audio recording 101 may be in a format that does not lend itself to optimal processing, such as FLAC or mp3 etc. The audio data 105 can then be processed in order to provide mono audio data 107 with a sample rate of 16kHz, for example. Using the audio data 107 as input to an STT engine, 109 STT data representing a transcript of the input user audio recording 101 can be generated. The audio data 107 can be processed using an audio cuts engine 111 to generate a set of audio cuts 115. That is, according to an example, a set of first audio samples are generated from user audio data 107, wherein each audio sample of the set of first audio samples comprises a segment of natural speech determined from the user audio data 107. A set of second audio samples are generated from the user audio data 107, wherein each audio sample of the set of second audio samples comprises a segment of audio representing a phoneme determined from the user audio data 107. As such, the audio cuts 115 comprise the set of first audio samples and the set of second audio samples. Audio cuts engine 111 can be used to generate metadata 113 corresponding to the set of first audio samples and to the set of second audio samples. Metadata 113 can comprise information such as start and end times of utterances etc., as described above.

The audio cuts 115 comprising the set of first audio samples and the set of second audio samples as well as the metadata 113 are input for an embeddings engine 117 configured to generate embeddings. In an example, the embeddings engine 117 is configured to generate, using the set of first audio samples, a set of first embeddings comprising respective vector representations for each audio sample of the set of first audio samples, and to generate, using the set of second audio samples, a set of second embeddings comprising respective vector representations for each audio sample of the set of second audio samples, altogether embeddings 119. Embeddings metadata 121 can be generated representing information associated with the set of first embeddings and/or the set of second embeddings.

As will be explained in more detail below, the set of first embeddings and the set of second embeddings are used to generate respective measures of probability for each symptom of a set of multiple symptoms for the user. For example, using the probability measures, the set of metrics representing indicators of the psychological state of the user can be generated. In the example of figure 1, the set of first embeddings and the set of second embeddings are input to a set of observable models 121 that generate the respective measures of probability for each symptom of a set of multiple symptoms for the user, which can be combined in order to create a set of wellness scores 125 as described above. The scores can be compared against benchmark data 127, and, as described above, the metadata 103 can be used to refine the benchmark data used. Finally, a score 129 comprising a set of scores 131 is generated, representing a final output metrics representing indicators of the psychological state of a user.

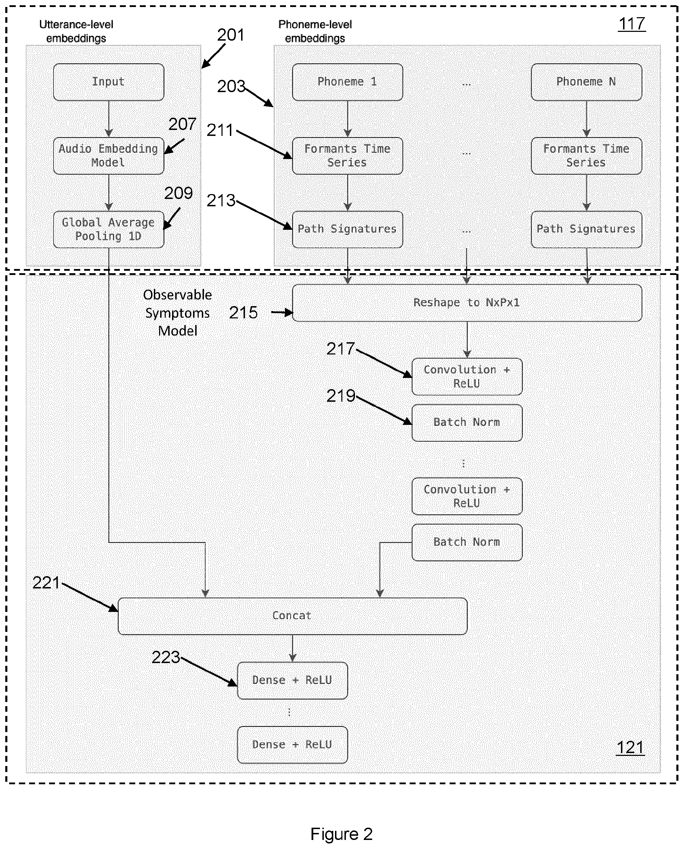

Figure 2 is a schematic representation of part of the system of figure 1, according to an example. In the example of figure 2, more detail for the embeddings engine 117 is depicted, along with more detail with respect to the data that is input from the embedding engine 117 to the models 121.

In an example, the embeddings engine 117 comprises two parts: the utterance-level embeddings engine 201 and the phoneme-level embeddings engine 203.

The definitions of Global Average Pooling 1D 209, Concat 221, Dense 223, ReLU217, 223, Convolution 217, Batch Norm 219, and Reshape 215 follow the standard definitions for Neural Networks layers. Implementations of these layers are offered by platforms such as Tensorflow, PyTorch or JAX and so on.

The utterance-level embeddings engine 201 takes a standardised input recording from the set of audio cuts 115 and processes it through an Audio Embedding Model 207, such as one of the models described above (TRILL, WAVE2VEC etc.), which produces as an output an array of embeddings 119; the Global Average Pooling 1D step 209 averages the array across the time dimension and outputs an utterance-level embedding 119 which can be fed to the downstream model Observable Symptoms Model 121.

The phoneme-level embeddings engine 203 takes as input a set of phonemes. The audio cuts 115 comprise pre-segmented phoneme-level audio cuts (as well as utterance level audio cuts, as described above). Accordingly, phoneme-level audio cuts can be input to the phoneme-level embeddings engine 203. For each phoneme-specific audio cut formants (F1 and F2, in particular) are extracted using the phoneme-level embeddings engine 203 and the formants time series that is produced is fed to a path signatures engine 213 which transforms the time series into a phoneme-level embedding ready to be consumed by the downstream model Observable Symptoms Model 121.

The Observable Symptoms Model 121 at first takes as input the output of the phoneme-level embeddings engine 203; 215 reshapes the input to form a tensor of dimensions NxPx1 where N is the number of phonemes and P the number of path signatures features. This reshaped tensor is fed to a collection of Neural Network blocks constituted by a sequence of Convolutional layers followed by ReLU activation functions 217 and Batch Norm layers 219. The last block's output gets Concatenated 221 with the output of the utterance level embeddings engine 201; after the concatenation a series of blocks composed of a Dense layer followed by a ReLU activation function 223 transforms the data. Appending a simple linear output layer at the end, all regression models can be completed, while appending a linear model with logistic activation function all classification models can be completed.

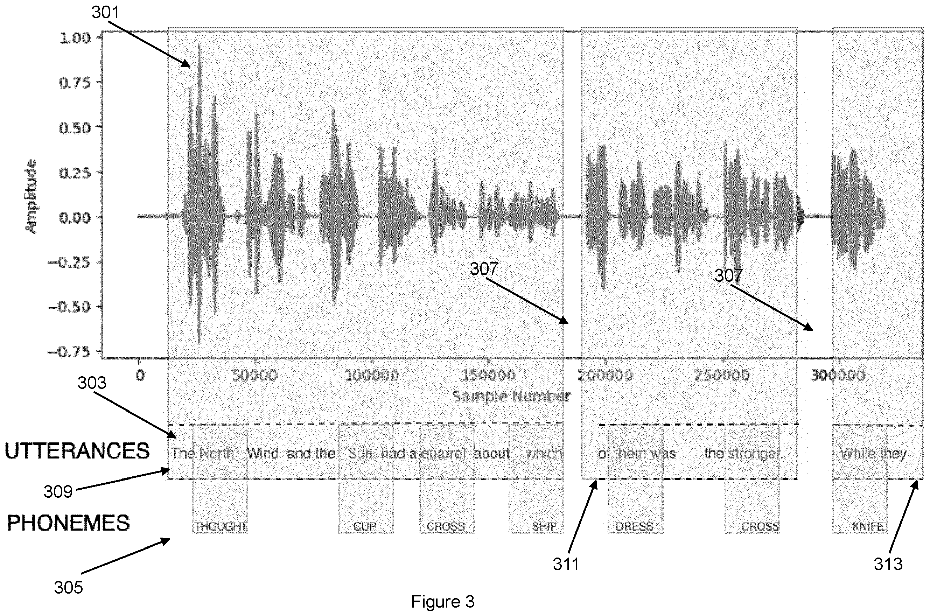

Figure 3 is a schematic representation of user audio data, according to an example. In the example of figure 3, the user audio data 107 comprises a recording of user speech from which utterances and phonemes can be determined. The waveform 301 comprises a mono 16kHz extract of user speech representing an audio recording of the user saying the words 303. Three utterances (309, 311 and 313) are determined on the basis of the periods of silence 307, as defined above, including an initial silence at the beginning of the waveform, in each case where the amplitude is below a preselected threshold amplitude value (and/or zero). Within these utterances, phonemes 305 can be provided, where a specific vowel phoneme for a word is referred to using its corresponding prototype word, as described above. Accordingly, 309, 311 and 313 represent three utterance cuts (i.e., three audio samples of the set of first audio samples from the user audio data), and the phonemes 305 represent multiple phonemes of the set of second audio samples from the user audio data.

Examples in the present disclosure can be provided as procedures, methods, systems or machine-readable instructions, such as any combination of software, hardware, firmware or the like. Such machine-readable instructions may be included on a computer readable storage medium (including but not limited to disc storage, CD-ROM, optical storage, etc.) having computer readable program codes therein or thereon.

The present disclosure is described with reference to flow charts and/or block diagrams of the method, devices and systems according to examples of the present disclosure. Although the flow diagrams described above show a specific order of execution, the order of execution may differ from that which is depicted. Blocks described in relation to one flow chart may be combined with those of another flow chart. In some examples, some blocks of the flow diagrams may not be necessary and/or additional blocks may be added. It shall be understood that each flow and/or block in the flow charts and/or block diagrams, as well as combinations of the flows and/or diagrams in the flow charts and/or block diagrams can be realized by machine readable instructions.

The machine-readable instructions may, for example, be executed by a machine such as an apparatus (e.g., an apparatus for generating a set of metrics representing indicators of the psychological state of a user), a general-purpose computer, a platform comprising user equipment such as a smart device, e.g., a smart phone, a special purpose computer, an embedded processor or processors of other programmable data processing devices to realize the functions described in the description and diagrams. In particular, a processor or processing apparatus may execute the machine-readable instructions. Thus, modules of apparatus (e.g. an embeddings engine and/or an STT engine, etc.) may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate set etc. The methods and modules may all be performed by a single processor or divided amongst several processors.

Such machine-readable instructions may also be stored in a computer readable storage that can guide the computer or other programmable data processing devices to operate in a specific mode. For example, the instructions may be provided on a non-transitory computer readable storage medium encoded with instructions, executable by a processor.

Further, the teachings herein may be implemented in the form of a computer or software product, such as a non-transitory machine-readable storage medium, the computer software or product being stored in a storage medium and comprising a plurality of instructions, e.g., machine readable instructions, for making a computer device implement the methods recited in the examples of the present disclosure.

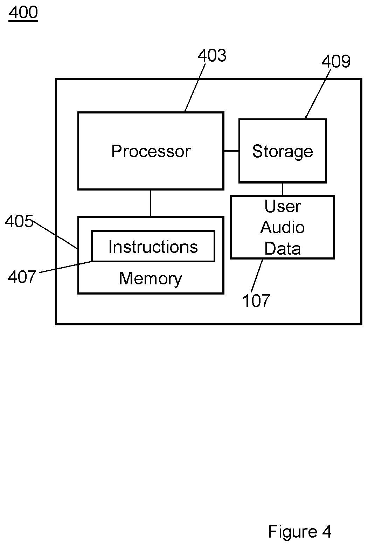

Figure 4 is a schematic representation of a machine according to an example. The machine of figure 4 can implement a system as described above with reference to figures 1 to 4, for example. The machine 400 comprises a processor 403, and a memory 405 to store instructions 407, executable by the processor 403. The machine comprises a storage 409 that can be used to store data representing, e.g., metadata, audio data, audio samples and so on, as described above. The instructions 407, executable by the processor 403, can cause the machine 400 to generate a set of first audio samples from user audio data, wherein each audio sample of the set of first audio samples comprises a segment of natural speech determined from the user audio data, generate a set of second audio samples from the user audio data, wherein each audio sample of the set of second audio samples comprises a segment of audio representing a phoneme determined from the user audio data, generate, using the set of first audio samples, a set of first embeddings comprising respective vector representations for each audio sample of the set of first audio samples, generate, using the set of second audio samples, a set of second embeddings comprising respective vector representations for each audio sample of the set of second audio samples, generate, using the set of first embeddings and the set of second embeddings, respective measures of probability for each symptom of a set of multiple symptoms for the user, and using the probability measures, calculating the set of metrics.

Accordingly, the machine 400 can implement a method for generating a set of metrics representing indicators of the psychological state of a user.

In some examples, some methods can be performed in a cloud-computing or network-based environment. Cloud-computing environments may provide various services and applications via the Internet. These cloud-based services (e.g., software as a service, platform as a service, infrastructure as a service, etc.) may be accessible through a web browser or other remote interface of the user equipment for example. Various functions described herein may be provided through a remote desktop environment or any other cloud-based computing environment.

While various embodiments have been described and/or illustrated herein in the context of fully functional computing systems, one or more of these exemplary embodiments may be distributed as a program product in a variety of forms, regardless of the particular type of computer-readable-storage media used to actually carry out the distribution. The embodiments disclosed herein may also be implemented using software modules that perform certain tasks. These software modules may include script, batch, or other executable files that may be stored on a computer-readable storage medium or in a computing system. In some embodiments, these software modules may configure a computing system to perform one or more of the exemplary embodiments disclosed herein. In addition, one or more of the modules described herein may transform data, physical devices, and/or representations of physical devices from one form to another.

The preceding description has been provided to enable others skilled in the art to best utilise various aspects of the exemplary embodiments disclosed herein. This exemplary description is not intended to be exhaustive or to be limited to any precise form disclosed. Many modifications and variations are possible without departing from the spirit and scope of the instant disclosure. The embodiments disclosed herein should be considered in all respects illustrative and not restrictive. Reference should be made to the appended claims and their equivalents in determining the scope of the instant disclosure.

## Claims

1. Apparatus for generating a set of metrics representing indicators of the psychological state of a user, the apparatus comprising:
a processor;
a memory coupled to the processor, the memory configured to store program code executable by the processor, the program code comprising one or more instructions, whereby to cause the apparatus to:
generate a set of first audio samples from user audio data, wherein each audio sample of the set of first audio samples comprises a segment of natural speech determined from the user audio data;
generate a set of second audio samples from the user audio data, wherein each audio sample of the set of second audio samples comprises a segment of audio representing a phoneme determined from the user audio data;
generate, using the set of first audio samples, a set of first embeddings comprising respective vector representations for each audio sample of the set of first audio samples;
generate, using the set of second audio samples, a set of second embeddings comprising respective vector representations for each audio sample of the set of second audio samples;
generate, using the set of first embeddings and the set of second embeddings, respective measures of probability for each symptom of a set of multiple symptoms for the user; and
using the probability measures, calculate the set of metrics.

2. The apparatus as claimed in claim 1, the program code comprising one or more instructions, whereby to cause the apparatus to:
generate a set of third embeddings by concatenating respective ones of the vector representations for each audio sample of the set of first audio samples with a corresponding vector representation for each audio sample of the set of second audio samples.

3. The apparatus as claimed in claim 1 or 2, the program code comprising one or more instructions, whereby to cause the apparatus to:
calculate, using the set of second audio samples, respective F1 formant time series and respective F2 formant time series for each phoneme determined from the user audio data; and
use the F1 formant time series and the F2 formant time series to generate a set of two dimensional path signatures for respective ones of the phonemes determined from the user audio data.

4. The apparatus as claimed in a preceding claim, the program code comprising one or more instructions, whereby to cause the apparatus to:
combine the probability measures according to a predefined set of profiles comprising additive and/or multiplicative rules for the probability measures.

5. The apparatus as claimed in a preceding claim, the program code comprising one or more instructions, whereby to cause the apparatus to:
compare respective measures of probability for each symptom of a set of multiple symptoms for the user with a set of benchmark values to generate a benchmarked probability score for each symptom.

6. A machine-readable storage medium encoded with instructions for generating a set of metrics representing indicators of the psychological state of a user, the instructions executable by a processor, whereby to cause the processor to:
generate a set of first audio samples from user audio data, wherein each audio sample of the set of first audio samples comprises a segment of natural speech determined from the user audio data;
generate a set of second audio samples from the user audio data, wherein each audio sample of the set of second audio samples comprises a segment of audio representing a phoneme determined from the user audio data;
generate, using the set of first audio samples, a set of first embeddings comprising respective vector representations for each audio sample of the set of first audio samples;
generate, using the set of second audio samples, a set of second embeddings comprising respective vector representations for each audio sample of the set of second audio samples;
generate, using the set of first embeddings and the set of second embeddings, respective measures of probability for each symptom of a set of multiple symptoms for the user; and
using the probability measures, calculate the set of metrics.

7. The machine-readable storage medium as claimed in claim 6, further comprising instructions executable by the processor, whereby to cause the processor to:
generate a set of third embeddings by concatenating respective ones of the vector representations for each audio sample of the set of first audio samples with a corresponding vector representation for each audio sample of the set of second audio samples.

8. The machine-readable storage medium as claimed in claim 6 or 7, further comprising instructions executable by the processor, whereby to cause the processor to:
calculate, using the set of second audio samples, respective F 1 formant time series and respective F2 formant time series for each phoneme determined from the user audio data; and
use the F 1 formant time series and the F2 formant time series to generate a set of two dimensional path signatures for respective ones of the phonemes determined from the user audio data.

9. The machine-readable storage medium as claimed in any of claims 6 to 8, further comprising instructions executable by the processor, whereby to cause the processor to:
combine the probability measures according to a predefined set of profiles comprising additive and/or multiplicative rules for the probability measures.

10. The machine-readable storage medium as claimed in any of claims 6 to 9, further comprising instructions executable by the processor, whereby to cause the processor to:
compare respective measures of probability for each symptom of a set of multiple symptoms for the user with a set of benchmark values to generate a benchmarked probability score for each symptom.

11. A method for generating a set of metrics representing indicators of the psychological state of a user, the method comprising:
generating a set of first audio samples from user audio data, wherein each audio sample of the set of first audio samples comprises a segment of natural speech determined from the user audio data;
generating a set of second audio samples from the user audio data, wherein each audio sample of the set of second audio samples comprises a segment of audio representing a phoneme determined from the user audio data;
generating, using the set of first audio samples, a set of first embeddings comprising respective vector representations for each audio sample of the set of first audio samples;
generating, using the set of second audio samples, a set of second embeddings comprising respective vector representations for each audio sample of the set of second audio samples;
generating, using the set of first embeddings and the set of second embeddings, respective measures of probability for each symptom of a set of multiple symptoms for the user; and
using the probability measures, calculating the set of metrics.

12. The method as claimed in claim 11, further comprising:
generating a set of third embeddings by concatenating respective ones of the vector representations for each audio sample of the set of first audio samples with a corresponding vector representation for each audio sample of the set of second audio samples.

13. The method as claimed in claim 11 or 12, further comprising:
calculating, using the set of second audio samples, respective F1 formant time series and respective F2 formant time series for each phoneme determined from the user audio data; and
using the F1 formant time series and the F2 formant time series, generating a set of two dimensional path signatures for respective ones of the phonemes determined from the user audio data.

14. The method as claimed in an any of claims 11 to 13, further comprising:
combining the probability measures according to a predefined set of profiles comprising additive and/or multiplicative rules for the probability measures.

15. The method as claimed in an any of claims 11 to 14, further comprising:
comparing respective measures of probability for each symptom of a set of multiple symptoms for the user with a set of benchmark values to generate a benchmarked probability score for each symptom.
